# EUROPÄISCHE PATENTANMELDUNG

(11) **EP 4 083 431 A1**
(43) Veröffentlichungstag der Anmeldung: **02.11.2022**
(21) Anmeldenummer: 22168230.5
(22) Anmeldetag: 13.04.2022
(51) Int. Cl.: F04D 13/06, F04D 29/041, F04D 29/048, H02K 7/09

(54) **ELEKTROMAGNETISCHER DREHANTRIEB, ZENTRIFUGALPUMPE UND PUMPENEINHEIT**

(30) Priorität: 26.04.2021 EP 21170397
(71) Anmelder: Levitronix GmbH, 8005 Zürich (CH)
(72) Erfinder: Steinert, Daniel, 8180 Bülach (CH); Schneeberger, Thomas, 3006 Bern (CH); Nussbaumer, Thomas, 8049 Zürich (CH); Stettler, Marcel, 5600 Lenzburg (CH); Giné, Jonas, 8404 Stadel (Winterthur) (CH)
(74) Vertreter: IPS Irsch AG

(57) **Zusammenfassung**

Es wird ein elektromagnetischer Drehantrieb vorgeschlagen, der als Innenläufer ausgestaltet ist, mit einem Rotor (3), der einen ring- oder scheibenförmigen magnetisch wirksamen Kern (31) umfasst, welcher von einem radial aussenliegend angeordneten Stator (2) umgeben ist, wobei der Stator (2) eine Mehrzahl von Statorpolen (21) aufweist, die um den magnetisch wirksamen Kern (31) herum angeordnet sind, und von denen jeder jeweils durch eine dem magnetisch wirksamen Kern (31) des Rotors (3) zugewandte Stirnfläche (211) begrenzt wird, wobei der Stator (2) als Lager- und Antriebsstator ausgestaltet ist, mit welchem der Rotor (3) im Betriebszustand berührungslos magnetisch um eine Solldrehachse antreibbar ist, die eine axiale Richtung (A) definiert, und mit welchem der Rotor (3) berührungslos magnetisch bezüglich des Stators (2) lagerbar ist, wobei der Rotor (3) in einer zur axialen Richtung (A) senkrechten radialen Ebene (E) aktiv magnetisch gelagert ist, und in axialer Richtung (A) sowie gegen Verkippungen passiv magnetisch stabilisiert ist. Der magnetisch wirksame Kern (31) des Rotors (3) weist eine Rotorhöhe (HR) auf, welche die maximale Erstreckung des magnetisch wirksamen Kerns (31) in axialer Richtung (A) ist, wobei die Rotorhöhe (HR) grösser ist als eine Statorpolhöhe (HS), welche durch die maximale Erstreckung der Stirnflächen (211) der Statorpole (21) in axialer Richtung (A) festgelegt ist. Durch die Erfindung werden ferner eine Zentrifugalpumpe mit einem solchen Drehantrieb (1) sowie eine Pumpeneinheit vorgeschlagen.

## Beschreibung

Die Erfindung betrifft einen elektromagnetischen Drehantrieb, eine Zentrifugalpumpe mit einem solchen elektromagnetischen Drehantrieb, sowie eine Pumpeneinheit für eine solche Zentrifugalpumpe gemäss dem Oberbegriff des unabhängigen Patentanspruchs der jeweiligen Kategorie.

Es sind elektromagnetische Drehantriebe bekannt, die nach dem Prinzip des lagerlosen Motors ausgestaltet sind und betrieben werden. Mit dem Begriff lagerloser Motor ist dabei ein elektromagnetischer Drehantrieb gemeint, bei welchem der Rotor vollkommen magnetisch bezüglich des Stators gelagert ist, wobei keine separaten magnetischen Lager vorgesehen sind. Der Stator ist dazu als Lager- und Antriebsstator ausgestaltet, der sowohl Stator des elektrischen Antriebs als auch Stator der magnetischen Lagerung ist. Mit den elektrischen Wicklungen des Stators lässt sich ein magnetisches Drehfeld erzeugen, welches zum einen ein Drehmoment auf den Rotor ausübt, das dessen Rotation um eine Solldrehachse bewirkt, und welches zum anderen eine beliebig einstellbare Querkraft auf den Rotor ausübt, sodass dessen radiale Position aktiv steuerbar bzw. regelbar ist. Somit sind drei Freiheitsgrade des Rotors aktiv regelbar, nämlich seine Rotation sowie seine radiale Position (zwei Freiheitsgrade). Bezüglich dreier weiterer Freiheitsgrade, nämlich seiner Position in axialer Richtung und Verkippungen bezüglich der zur Solldrehachse senkrechten radialen Ebene (zwei Freiheitsgrade), ist der Rotor passiv magnetisch, das heisst nicht ansteuerbar, durch Reluktanzkräfte gelagert bzw. stabilisiert. Das Nichtvorhandensein eines separaten magnetischen Lagers bei vollständiger magnetischer Lagerung des Rotors ist die Eigenschaft, welcher der lagerlose Motor seinen Namen verdankt. In dem Lager- und Antriebsstator lässt sich die Lagerfunktion nicht von der Antriebsfunktion separieren.

Ein solcher lagerloser Motor, der beispielsweise in der US 6,053,705 offenbart wird, hat sich in einer Vielzahl von Anwendungen bewährt. Aufgrund der Abwesenheit von mechanischen Lagern eignet sich der lagerlose Motor insbesondere für Pump-, Misch- oder Rührvorrichtungen, mit denen sehr empfindliche Substanzen gefördert werden, beispielsweise Blutpumpen, oder bei denen sehr hohe Anforderungen an die Reinheit gestellt werden, beispielsweise in der pharmazeutischen Industrie oder in der biotechnologischen Industrie, oder mit denen abrasive oder aggressive Substanzen gefördert werden, welche mechanische Lager sehr schnell zerstören würden, beispielsweise Pumpen oder Mischer für Slurry in der Halbleiterindustrie.

Ein weiterer Vorteil des Prinzips des lagerlosen Motors ergibt sich bei der Ausgestaltung des Rotors als Integralrotor, der sowohl der Rotor des elektromagnetischen Drehantriebs ist, als auch der Rotor der Pumpe. Neben der berührungslosen magnetischen Lagerung resultiert hier der Vorteil einer sehr kompakten und platzsparenden Ausgestaltung.

Zudem erlaubt das Prinzip des lagerlosen Motors auch Ausgestaltungen, beispielsweise von Zentrifugalpumpen, bei denen der Rotor sehr leicht vom Stator trennbar ist. Dies ist ein sehr grosser Vorteil, weil damit beispielsweise der Rotor bzw. die Pumpeneinheit, welche den Rotor umfasst, als Einmalteil für den Einmalgebrauch ausgestaltet werden kann. Solche Einmalanwendungen ersetzen heute häufig Prozesse, bei denen früher aufgrund der sehr hohen Reinheitsanforderungen alle diejenigen Komponenten, welche im Prozess mit den zu behandelnden Substanzen in Kontakt kommen, aufwändig gereinigt und sterilisiert werden müssen, beispielsweise mittels Dampfsterilisierung. Bei der Ausgestaltung für den Einmalgebrauch werden diejenigen Komponenten, welche mit den zu behandelnden Substanzen in Kontakt kommen, nur genau einmal verwendet und dann bei der nächsten Anwendung durch neue, das heisst ungebrauchte, Einmalteile ersetzt.

Als Beispiele seien hier die Pharmaindustrie und die biotechnologische Industrie genannt. Hier werden häufig Lösungen und Suspensionen hergestellt, die eine sorgfältige und schonende Förderung von Substanzen verlangen.

In der Pharmaindustrie müssen beispielsweise bei der Herstellung von pharmazeutisch wirksamen Substanzen höchste Ansprüche an die Reinheit gestellt werden, oft müssen die mit den Substanzen in Kontakt kommenden Komponenten sogar steril sein. Ähnliche Anforderungen ergeben sich auch in der Biotechnologie, beispielsweise bei der Herstellung, Behandlung oder Züchtung von biologischen Substanzen, Zellen oder Mikroorganismen, wo ein extrem hohes Mass an Reinheit gewährleistet sein muss, um die Brauchbarkeit des hergestellten Produkts nicht zu gefährden. Als ein weiteres Beispiel seien hier Bioreaktoren genannt, in denen beispielsweise biologische Substitute für Gewebe oder spezielle Zellen oder andere sehr empfindliche Substanzen gezüchtet werden. Auch hier benötigt man Zentrifugalpumpen, um beispielsweise eine kontinuierliche Durchmischung der Nährflüssigkeit beziehungsweise deren kontinuierliche Zirkulation im Mischbehälter zu gewährleisten. Dabei muss eine sehr hohe Reinheit gewährleistet sein, um die Substanzen oder die erzeugten Produkte vor Kontaminationen zu schützen. Ein weiteres Anwendungsbeispiel sind Blutpumpen, bei denen natürlich höchste Anforderungen an die Reinheit und zudem an die schonende Behandlung insbesondere der roten Blutkörperchen gestellt werden.

Bei solchen Anwendungen, bei denen eine Zentrifugalpumpe für den Einmalgebrauch ausgelegt ist, setzt sich die Zentrifugalpumpe typischerweise aus einer Einmalvorrichtung und einer wiederverwendbaren Vorrichtung zusammen. Dabei umfasst die Einmalvorrichtung diejenigen Komponenten, die mit den Substanzen in Kontakt kommen, und die als Einmal (single-use) -Teile für den Einmalgebrauch ausgestaltet sind. Dies ist beispielsweise die Pumpeneinheit mit dem Pumpengehäuse und dem darin angeordneten Rotor, welcher das Flügelrad der Zentrifugalpumpe bildet. Die wiederverwendbare Vorrichtung umfasst diejenigen Komponenten, die dauerhaft also mehrfach verwendet werden, beispielsweise den Stator des elektromagnetischen Drehantriebs.

Bei alle diesen Anwendungen, bei denen der lagerlose Motor erfolgreich eingesetzt wird, ist es prinzipiell möglich, den lagerlosen Motor als Innenläufer, das heisst mit innenliegendem Rotor und darum herum angeordneten Stator auszugestalten. Allerdings besteht bei solchen elektromagnetischen Drehantrieben, wie sie beispielsweise in der US 6,053,705 offenbart werden, noch Verbesserungsbedarf.

So weisen derartige Vorrichtungen üblicherweise eine relativ geringe axiale Steifigkeit der magnetischen Lagerung des Rotors auf, insbesondere auch deshalb, weil die Flussdichte des magnetischen Flusses im Luftspalt zwischen dem Stator und dem Rotor eher klein ist, und der Gradient der magnetischen Flussdichte bei Auslenkungen des Rotors in der axialen Richtung aufgrund der hohen Streuung auch eher klein ist.

Ausgehend von diesem Stand der Technik ist es daher eine Aufgabe der Erfindung, einen elektromagnetischen Drehantrieb vorzuschlagen, der als Innenläufer ausgestaltet ist, und der einen magnetisch berührungslos antreibbaren sowie magnetisch berührungslos lagerbaren Rotor umfasst, wobei insbesondere die axiale Steifigkeit der magnetischen Stabilisierung des Rotors deutlich verbessert ist. Zudem ist es eine Aufgabe der Erfindung, eine Zentrifugalpumpe vorzuschlagen, die einen solchen Drehantrieb umfasst. Ferner soll durch die Erfindung eine Pumpeneinheit für eine solche Zentrifugalpumpe vorgeschlagen werden, die insbesondere auch für den Einmalgebrauch ausgestaltet werden kann.

Die diese Aufgabe lösenden Gegenstände der Erfindung sind durch die Merkmale des unabhängigen Patentanspruchs der jeweiligen Kategorie gekennzeichnet.

Erfindungsgemäss wird also ein elektromagnetischer Drehantrieb vorgeschlagen, der als Innenläufer ausgestaltet ist, mit einem Rotor, der einen ring- oder scheibenförmigen magnetisch wirksamen Kern umfasst, welcher von einem radial aussenliegend angeordneten Stator umgeben ist, wobei der Stator eine Mehrzahl von Statorpolen aufweist, die um den magnetisch wirksamen Kern herum angeordnet sind, und von denen jeder jeweils durch eine dem magnetisch wirksamen Kern des Rotors zugewandte Stirnfläche begrenzt wird, wobei der Stator als Lager- und Antriebsstator ausgestaltet ist, mit welchem der Rotor im Betriebszustand berührungslos magnetisch um eine Solldrehachse antreibbar ist, die eine axiale Richtung definiert, und mit welchem der Rotor berührungslos magnetisch bezüglich des Stators lagerbar ist, wobei der Rotor in einer zur axialen Richtung senkrechten radialen Ebene aktiv magnetisch gelagert ist, und in axialer Richtung sowie gegen Verkippungen passiv magnetisch stabilisiert ist. Der magnetisch wirksame Kern des Rotors weist eine Rotorhöhe auf, welche die maximale Erstreckung des magnetisch wirksamen Kerns in axialer Richtung ist, wobei die Rotorhöhe grösser ist als eine Statorpolhöhe, welche durch die maximale Erstreckung der Stirnflächen der Statorpole in axialer Richtung festgelegt ist.

Besonders bevorzugt ist dabei der magnetisch wirksame Kern des Rotors permanentmagnetisch ausgestaltet, das heisst er umfasst mindestens einen Permanentmagneten oder er besteht aus einem permanentmagnetischen Material. Ferner ist es bevorzugt, dass jede Stirnfläche der Statorpole in der axialen Richtung die gleiche Erstreckung hat, sodass für jede Stirnfläche ihre jeweilige Erstreckung in axialer Richtung gleich der Statorpolhöhe ist.

Dadurch, dass der magnetisch wirksame Kern des Rotors die Rotorhöhe aufweist, die grösser ist als die Statorpolhöhe, resultiert eine Konzentration des magnetischen Flusses im Luftspalt zwischen den Stirnflächen und dem magnetisch wirksamen Kern des Rotors, das heisst die magnetische Flussdichte nimmt im Luftspalt zu. Da die axiale Steifigkeit der magnetischen Stabilisierung des Rotors zumindest näherungsweise quadratisch mit der magnetischen Flussdichte im Luftspalt zunimmt, nimmt die axiale Steifigkeit überproportional mit der Rotorhöhe zu. Somit resultiert daraus, dass die Rotorhöhe grösser ist als die Statorpolhöhe eine Vergrösserung der axialen Steifigkeit der magnetischen Lagerung bzw. Stabilisierung des Rotors.

Um die Kippsteifigkeit des Rotors, also seinen Widerstand gegen Verkippungen relativ zu der radialen Ebene, die senkrecht zur axialen Richtung liegt, nicht zu stark zu reduzieren, ist es bevorzugt, dass das Verhältnis aus dem Aussendurchmesser des magnetisch wirksamen Kerns des Rotors und der Rotorhöhe den Wert von 2.8 nicht unterschreitet.

Bei einer bevorzugten Ausführungsform umfasst der magnetisch wirksame Kern einen zentralen Bereich, welcher bezüglich der axialen Richtung zwischen einem ersten Randbereich und einem zweiten Randbereich angeordnet ist, und welcher einen Rotordurchmesser aufweist, wobei der erste Randbereich eine erste axiale Begrenzungsfläche des magnetisch wirksamen Kerns bildet, die einen ersten Randdurchmesser aufweist, wobei der zweite Randbereich eine zweite axiale Begrenzungsfläche des magnetisch wirksamen Kerns bildet, die einen zweiten Randdurchmesser aufweist, und wobei jeder Randdurchmesser kleiner ist als der Rotordurchmesser.

Bei dieser Ausführungsform kann somit der radial aussenliegende Bereich des magnetischen Kerns des Rotors mit einer geringeren Höhe - gemessen in der axialen Richtung - ausgestaltet werden, als der Bereich zwischen der ersten und der zweiten axialen Begrenzungsfläche. Dies hat den Vorteil, dass ebenfalls eine Vergrösserung der axialen Steifigkeit der magnetischen Stabilisierung resultiert, aber eine deutlich geringere Abnahme der Kippsteifigkeit.

Vorzugsweise weist der zentrale Bereich eine zentrale Höhe auf, welches die Erstreckung des zentralen Bereichs in axialer Richtung ist, wobei die zentrale Höhe gleich gross ist wie die Statorpolhöhe. Dies hat den Vorteil, dass der radial aussenliegende Bereich des magnetisch wirksamen Kerns des Rotors, also derjenige Bereich, welcher den Stirnflächen der Statorpole direkt gegenüber liegt, in der axialen Richtung die gleiche Erstreckung hat wie die Stirnflächen der Statorpole, sodass im Luftspalt eine sehr hohe magnetische Flussdichte resultiert, was auch im Hinblick auf das Drehmoment vorteilhaft ist, welches die Rotation des Rotors antreibt.

Eine weitere bevorzugte Massnahme ist es, dass der magnetisch wirksame Kern eine Aussenfläche aufweist, die weder zwischen dem zentralen Bereich und der ersten axialen Begrenzungsfläche noch zwischen dem zentralen Bereich und der zweiten axialen Begrenzungsfläche parallel zur axialen Richtung verläuft. Hierdurch lässt es sich realisieren, dass die Feldlinien des magnetischen Flusses zwischen dem zentralen Bereich und den beiden axialen Begrenzungsflächen nicht senkrecht zur axialen Richtung aus dem magnetisch wirksamen Kern des Rotors austreten, sondern unter einem Winkel der kleiner ist als 90°. Hierdurch treten die Feldlinien bezüglich der radialen Richtung weiter aussen in die Statorpole ein, wodurch sich die magnetische Lagerung bzw. Stabilisierung des Rotors verbessert.

Besonders bevorzugt ist es, dass mindestens einer des ersten und des zweiten Randbereichs in Form eines Kegelstumpfs oder in Form einer Kugelscheibe oder in Form einer Paraboloidscheibe ausgestaltet ist. Durch solche Ausgestaltungen lässt es sich insbesondere realisieren, dass die Aussenfläche, welche den magnetisch wirksamen Kern des Rotors begrenzt, sowohl zwischen dem zentralen Bereich und der ersten axialen Begrenzungsfläche als auch zwischen dem zentralen Bereich und der zweiten axialen Begrenzungsfläche nicht parallel zur axialen Richtung verläuft, sodass hier die Feldlinien des magnetischen Flusses unter einem von 90° verschiedenen Winkel aus dem magnetisch wirksamen Kern des Rotors austreten bzw. in diesen eintreten.

Der erste und der zweite Randbereich können unterschiedlich ausgestaltet sein. So kann beispielsweise der erste Randbereich kegelstumpfförmig ausgestaltet sein und der zweite Randbereich in Form einer Kugelscheibe. Natürlich ist es auch möglich, dass der erste Randbereich und der zweite Randbereich gleichartig ausgestaltet sind.

Gemäss einem ersten bevorzugten Ausführungsbeispiel, das auch als Radialmotor bezeichnet wird, trägt jeder Statorpol mindestens eine konzentrierte Wicklung, sodass jede konzentrierte Wicklung in der radialen Ebene angeordnet ist. Bei diesem ersten Ausführungsbeispiel sind also die Wicklungen zum Erzeugen der elektromagnetischen Felder in der gleichen Ebene angeordnet wie der magnetisch wirksame Kern des Rotors.

Gemäss einem zweiten bevorzugten Ausführungsbeispiel ist der Drehantrieb als Tempelmotor ausgestaltet, und der Stator weist eine Mehrzahl von Spulenkernen auf, von denen jeder einen stabförmigen Längsschenkel umfasst, welcher sich von einem ersten Ende in axialer Richtung bis zu einem zweiten Ende erstreckt, sowie einen Querschenkel, welcher an dem zweiten Ende des Längsschenkels und in der radialen Ebene angeordnet ist, und welcher sich in einer radialen Richtung erstreckt, die senkrecht zu der axialen Richtung ist, wobei jeder Querschenkel einen der Statorpole bildet, und wobei an jedem Längsschenkel mindestens eine konzentrierte Wicklung angeordnet ist, welche den jeweiligen Längsschenkel umgibt. Bei dieser Ausgestaltung als Tempelmotor sind also die Wicklungen zur Erzeugung der elektromagnetischen Felder unterhalb der magnetischen Mittelebene angeordnet und so ausgerichtet, dass ihre Spulenachse jeweils in axialer Richtung liegt.

Durch die Erfindung wird ferner eine Zentrifugalpumpe zum Fördern eines Fluids vorgeschlagen, die dadurch gekennzeichnet ist, dass die Zentrifugalpumpe einen elektromagnetischen Drehantrieb umfasst, der erfindungsgemäss ausgestaltet ist, wobei der Rotor des elektromagnetischen Drehantriebs als Rotor der Zentrifugalpumpe ausgestaltet ist.

Vorzugsweise umfasst die Zentrifugalpumpe eine Pumpeneinheit mit einem Pumpengehäuse, das einen Einlass und einen Auslass für das zu fördernde Fluid umfasst, wobei der Rotor im Pumpengehäuse angeordnet ist und eine Mehrzahl von Flügeln zum Fördern des Fluids umfasst, wobei die Pumpeneinheit derart ausgestaltet ist, dass die Pumpeneinheit so in den Stator einsetzbar ist, dass der magnetisch wirksame Kern des Rotors von den Statorpolen umgeben wird.

Ferner wird durch die Erfindung eine Pumpeneinheit für eine Zentrifugalpumpe vorgeschlagen, welche dadurch gekennzeichnet ist, dass die Pumpeneinheit für eine Zentrifugalpumpe ausgestaltet ist, welche gemäss der Erfindung ausgestaltet ist.

Gemäss einer bevorzugten Ausgestaltung umfasst das Pumpengehäuse ein Basisteil und einen Deckel, welche dichtend miteinander verbunden sind, wobei der Auslass des Pumpengehäuses vollständig im Basisteil angeordnet ist.

Auch ist es eine bevorzugte Massnahme, dass der Rotor eine zentrale Bohrung aufweist, welche sich in axialer Richtung vollständig durch den Rotor hindurch erstreckt, sodass der durch die Flügel generierte Axialschub zumindest teilweise kompensiert wird.

Vorzugsweise ist die Pumpeneinheit zum lösbaren Verbinden mit dem Stator der erfindungsgemässen Zentrifugalpumpe ausgestaltet.

Gemäss einer bevorzugten Ausgestaltung ist die Pumpeneinheit als Einmalvorrichtung für den Einmalgebrauch ausgestaltet.

Weitere vorteilhafte Massnahmen und Ausgestaltungen der Erfindung ergeben sich aus den abhängigen Ansprüchen.

Im Folgenden wird die Erfindung anhand von Ausführungsbeispielen und anhand der Zeichnung näher erläutert. In der teilweise schematischen Zeichnung zeigen:
- Fig. 1:: ein erstes Ausführungsbeispiel eines erfindungsgemässen elektromagnetischen Drehantriebs in einem Schnitt in axialer Richtung, wobei der Schnitt entlang der Schnittlinie I-I in Fig. 2 erfolgt,
- Fig. 2:: einen Schnitt durch das erste Ausführungsbeispiel in einem Schnitt senkrecht zur axialen Richtung entlang der Schnittlinie II-II in Fig. 1,
- Fig. 3:: wie Fig. 1, jedoch mit verkipptem Rotor,
- Fig. 4:: wie Fig. 1, jedoch mit in axialer Richtung verschobenem Rotor,
- Fig. 5:: den magnetisch wirksamen Kern des Rotors des ersten Ausführungsbeispiels,
- Fig. 6:: eine erste Variante für den magnetisch wirksamen Kern des Rotors in einer zu Fig. 1 analogen Darstellung,
- Fig. 7:: eine zweite Variante für den magnetisch wirksamen Kern des Rotors in einem Schnitt in axialer Richtung.
- Fig. 8:: eine dritte Variante für den magnetisch wirksamen Kern des Rotors,
- Fig. 9:: eine vierte Variante für den magnetisch wirksamen Kern des Rotors,
- Fig. 10:: ein zweites Ausführungsbeispiel eines erfindungsgemässen elektromagnetischen Drehantriebs in einem Schnitt in axialer Richtung,
- Fig. 11:: eine Aufsicht auf das zweite Ausführungsbeispiel aus der axialen Richtung,
- Fig. 12:: ein Ausführungsbeispiel einer erfindungsgemässen Zentrifugalpumpe in einem Schnitt in axialer Richtung,
- Fig. 13:: die Pumpeneinheit der Zentrifugalpumpe aus Fig. 12 in einem axialen Schnitt entlang der Schnittlinie XIII-XIII in Fig. 14, und
- Fig. 14:: eine Aufsicht auf die Pumpeneinheit aus Fig. 13 aus der axialen Richtung.

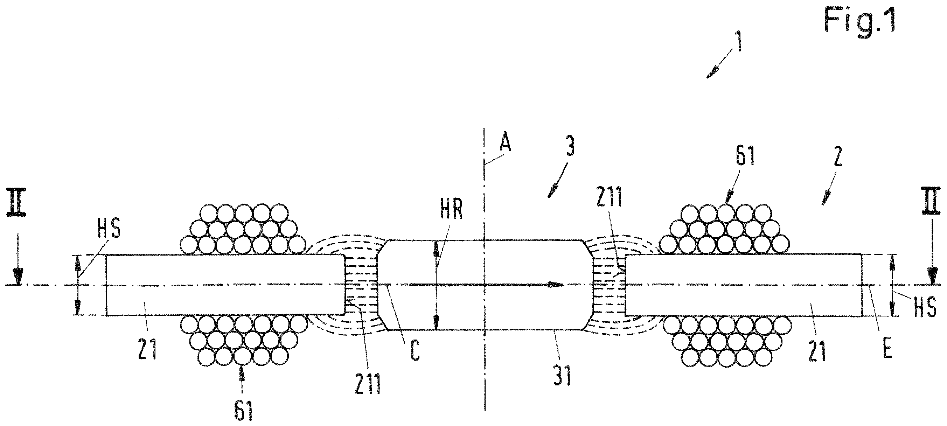

Fig. 1 zeigt eine perspektivische Schnittdarstellung eines ersten Ausführungsbeispiel eines erfindungsgemässen Drehantriebs, der gesamthaft mit dem Bezugszeichen 1 bezeichnet ist.

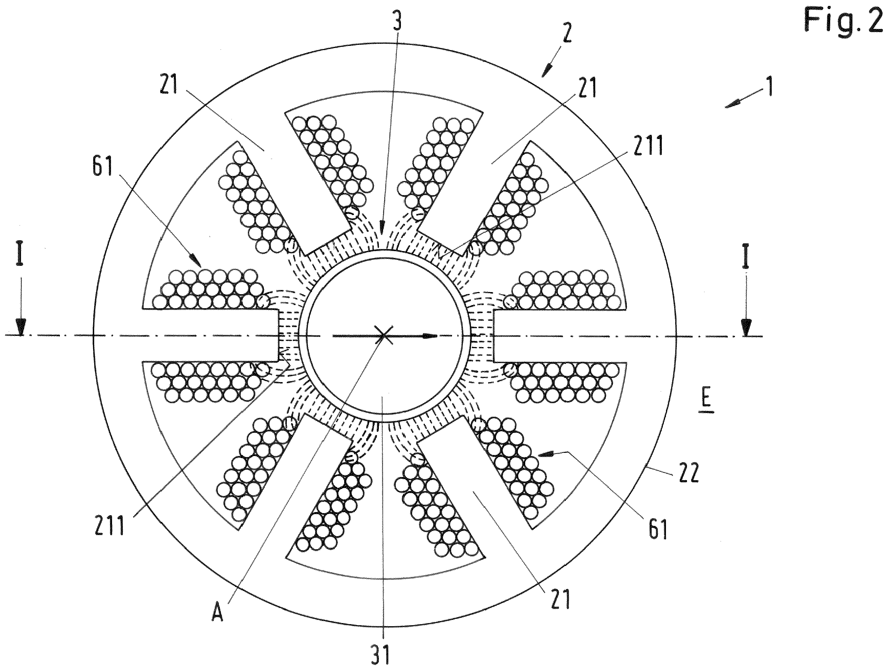

Fig. 1 zeigt den elektromagnetischen Drehantrieb 1 in einem Schnitt in axialer Richtung, wobei der Schnitt entlang der Schnittlinie I-I in Fig. 2 erfolgt. Zum besseren Verständnis zeigt
Fig. 2 noch einen Schnitt durch den elektromagnetischen Drehantrieb 1 in einem Schnitt senkrecht zur axialen Richtung A entlang der Schnittlinie II-II in Fig. 1.

Der elektromagnetische Drehantrieb 1 ist als Innenläufer ausgestaltet und umfasst einen Stator 2 sowie einen bezüglich des Stators 2 berührungslos magnetisch gelagerten Rotor 3. Ferner ist der Rotor 3 mittels des Stators 2 berührungslos magnetisch zur Rotation um eine Solldrehachse antreibbar. Die Solldrehachse bezeichnet dabei diejenige Achse, um welche sich der Rotor 3 im Betriebszustand dreht, wenn sich der Rotor 3 bezüglich des Stators 2 in einer zentrierten und unverkippten Lage befindet, so wie dies in Fig. 1 dargestellt ist. Diese Solldrehachse definiert eine axiale Richtung A. Üblicherweise stimmt die die axiale Richtung A festlegende Solldrehachse mit der Mittelachse des Stators 2 überein.

Im Folgenden wird mit einer radialen Richtung eine Richtung bezeichnet, welche senkrecht auf der axialen Richtung A steht.

Der Rotor 3 umfasst einen magnetisch wirksamen Kern 31, welcher ring- oder scheibenförmig ausgestaltet ist. Der magnetisch wirksame Kern 31 ist gemäss der Darstellung in Fig. 1 als Scheibe ausgestaltet und definiert eine magnetische Mittelebene C. Mit der magnetischen Mittelebene C des magnetisch wirksamen Kerns 31 des Rotors 3 wird diejenige Ebene senkrecht zur axialen Richtung A bezeichnet, in welcher der magnetisch wirksame Kern 31 des Rotors 3 im Betriebszustand gelagert wird, wenn der Rotor 3 nicht verkippt und in axialer Richtung A nicht ausgelenkt ist. In der Regel ist bei einem scheibenförmigen oder ringförmigen magnetisch wirksamen Kern 31 die magnetische Mittelebene C die geometrische Mittelebene des magnetisch wirksamen Kerns 31 des Rotors 3, die senkrecht zur axialen Richtung A liegt. Diejenige Ebene, in welcher der magnetisch wirksame Kern 31 des Rotors 3 im Betriebszustand im Stator 2 gelagert ist, wird auch als radiale Ebene E bezeichnet. Die radiale Ebene definiert die x-y-Ebene eines kartesischen Koordinatensystems, dessen z-Achse in axialer Richtung A verläuft. Ist der magnetisch wirksame Kern 31 des Rotors 3 nicht verkippt und bezüglich der axialen Richtung (A) nicht ausgelenkt, so stimmt die radiale Ebene E mit der magnetischen Mittelebene C überein.

Fig. 2 zeigt einen Schnitt, der in der magnetischen radialen Ebene E erfolgt.

Mit der radialen Position des magnetisch wirksamen Kerns 31 bzw. des Rotors 3 wird die Lage des Rotors 3 in der radialen Ebene E bezeichnet.

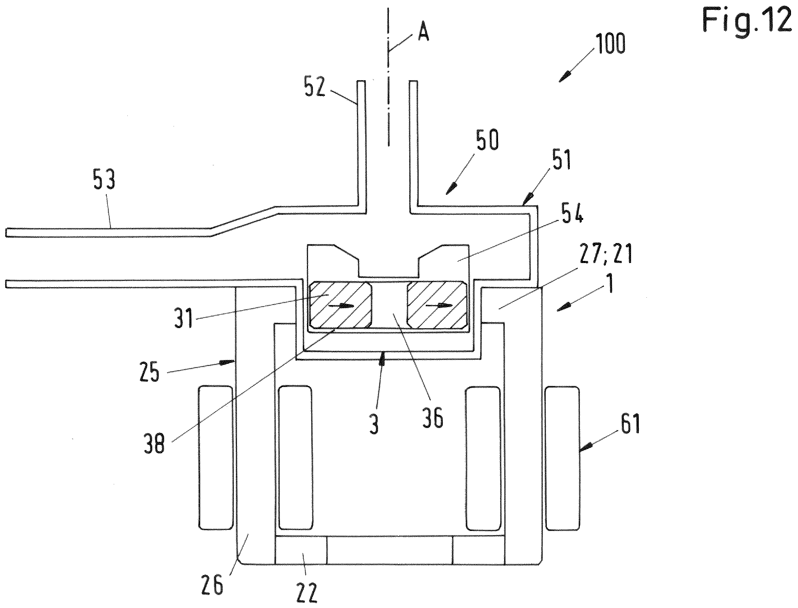

Da es für das Verständnis der Erfindung ausreichend ist, ist in der Zeichnung, z. B. in den Fig. 1-10, von dem Rotor 3 jeweils nur der magnetisch wirksame Kern 31 dargestellt. Es versteht sich, dass der Rotor 3 natürlich auch noch weitere Komponenten umfassen kann wie beispielsweise Ummantelungen oder Kapselungen, die vorzugsweise aus einem Kunststoff hergestellt sind, oder aus einem Metall oder aus einer Metalllegierung oder aus einer Keramik bzw. einem keramischen Werkstoff. Ferner kann der Rotor 3 auch Flügel zum Mischen, Rühren oder Pumpen von Fluiden (siehe z. B. Fig. 12) oder sonstige Komponenten umfassen.

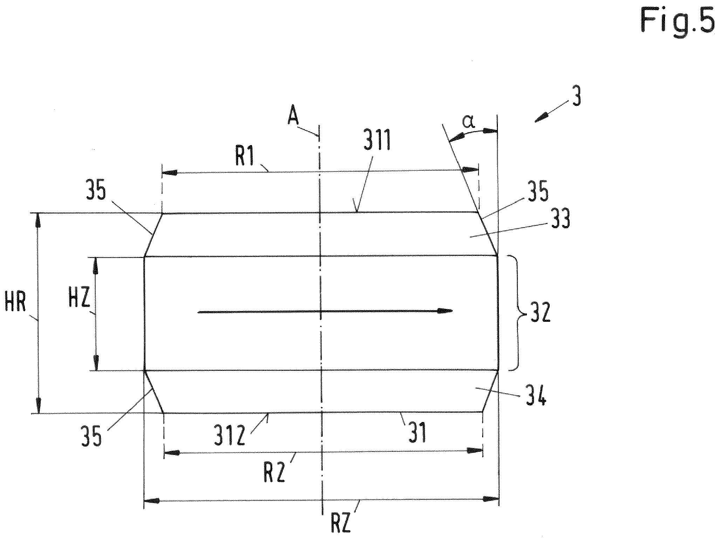

Zum besseren Verständnis zeigt Fig. 5 noch eine Ansicht des magnetisch wirksamen Kerns 31 des Rotors 3.

Wie dies bei einem Innenläufer üblich ist, ist der Rotor 3 und insbesondere der magnetisch wirksame Kern 31 des Rotors 3 von dem radial aussenliegend angeordneten Stator 2 umgeben. Der Stator 2 umfasst eine Mehrzahl von ausgeprägten Statorpolen 21- hier sechs Statorpole 21 -, die sich jeweils von einem radial aussenliegenden ringförmigen Rückschluss 22 in radialer Richtung nach innen auf den Rotor 3 hin erstrecken. Jeder Statorpol 21 ist in der radialen Ebene E angeordnet und wird jeweils durch eine dem magnetisch wirksamen Kern 31 des Rotors 3 zugewandte Stirnfläche 211 begrenzt. Während des Betriebs des elektromagnetischen Drehantriebs 1 ist es die Soll-Position, dass der magnetisch wirksame Kern 31 zwischen den Stirnflächen 211 der Statorpole 21 zentriert ist.

Um die für den magnetischen Antrieb und die magnetische Lagerung des Rotors 3 notwendigen elektromagnetischen Drehfelder zu erzeugen, tragen die Statorpole 21 Wicklungen. Bei dem hier beschriebenen Ausführungsbeispiel sind die Wicklungen als konzentrierte Wicklungen 61 beispielsweise so ausgestaltet, dass um jeden Statorpol 21 herum jeweils genau eine konzentrierte Wicklung 61 gewickelt ist, sodass auch jede konzentrierte Wicklung 61 in der radialen Ebene E angeordnet ist. Mit diesen konzentrierten Wicklungen 61 werden im Betriebszustand diejenigen elektromagnetischen Drehfelder erzeugt, mit welchen ein Drehmoment auf den Rotor 3 bewirkt wird, und mit welchen eine beliebig einstellbare Querkraft in radialer Richtung auf den Rotor 3 ausübbar ist, sodass die radiale Position des Rotors 3, also seine Position in der zur axialen Richtung A senkrechten radialen Ebene E, aktiv steuerbar bzw. regelbar ist.

Mit dem "magnetisch wirksamen Kern 31" des Rotors 3 ist derjenige Bereich des Rotors 3 gemeint, welcher für die Drehmomentbildung sowie für die Erzeugung der magnetischen Lagerkräfte magnetisch mit den Statorpolen 21 zusammenwirkt.

Wie bereits erwähnt, ist der magnetisch wirksame Kern 31 scheibenförmig ausgestaltet. Ferner ist der magnetisch wirksame Kern 31 permanentmagnetisch ausgestaltet. Dazu kann der magnetisch wirksame Kern 31 mindestens einen Permanentmagneten, aber auch mehrere Permanentmagnete umfassen oder-wie im hier beschriebenen Ausführungsbeispiel - vollständig aus einem permanentmagnetischen Material bestehen, sodass der magnetisch wirksame Kern 31 der Permanentmagnet ist. Die Magnetisierung des magnetisch wirksamen Kerns des Rotors 3 ist in Fig. 1, Fig. 2 und Fig. 5 jeweils durch den Pfeil ohne Bezugszeichen im magnetisch wirksamen Kern 31 dargestellt. Der magnetisch wirksame Kern 31 ist also in radialer Richtung magnetisiert.

Als Permanentmagnete bezeichnet man üblicherweise solche ferromagnetischen oder ferrimagnetischen Werkstoffe, die hartmagnetisch sind, also eine hohe Koerzitivfeldstärke aufweisen. Die Koerzitivfeldstärke ist diejenige magnetische Feldstärke, die man benötigt, um einen Stoff zu entmagnetisieren. Im Rahmen dieser Anmeldung wird unter einem Permanentmagneten ein Werkstoff bzw. ein Material verstanden, der eine Koerzitivfeldstärke, genauer gesagt eine Koerzitivfeldstärke der magnetischen Polarisation aufweist, die mehr als 10'000 A/m beträgt.

Sowohl der ringförmige Rückschluss 22 als auch die Statorpole 21 des Stators 2 sind jeweils aus einem weichmagnetischen Material gefertigt, weil sie als Flussleitelemente zur Führung des magnetischen Flusses dienen. Geeignete weichmagnetische Materialien sind beispielsweise ferromagnetische oder ferrimagnetische Materialien, also insbesondere Eisen, Nickel-Eisen, Kobalt-Eisen Silizium-Eisen oder Mu-Metall. Hierbei ist für den Stator 2, eine Ausgestaltung als Statorblechpaket bevorzugt, bei welcher die Statorpole 21 und der Rückschluss 22, geblecht ausgestaltet sind, das heisst sie bestehen aus mehreren dünnen Blech-Elementen, die gestapelt sind. Ferner ist es möglich, dass die Statorpole 21 und der Rückschluss 22 aus gepressten und anschliessend gesinterten Körnern der genannten Materialien bestehen. Die metallischen Körner sind dabei vorzugsweise in eine Kunststoffmatrix eingebettet, sodass sie zumindest teilweise gegeneinander isoliert sind, wodurch sich Wirbelstromverluste minimieren lassen. Es sind also für den Stator auch weichmagnetische Verbundwerkstoffe geeignet, welche aus elektrisch isolierten und zusammengepressten Metallpartikeln bestehen. Insbesondere können diese weichmagnetischen Verbundwerkstoffe, die auch als SMC (Soft Magnetic Composites) bezeichnet werden, aus Eisenpulverpartikeln bestehen, die mit einer elektrisch isolierenden Schicht beschichtet sind. Diese SMC werden dann mittels pulvermetallurgischer Verfahren zu der gewünschten Ausgestaltung geformt.

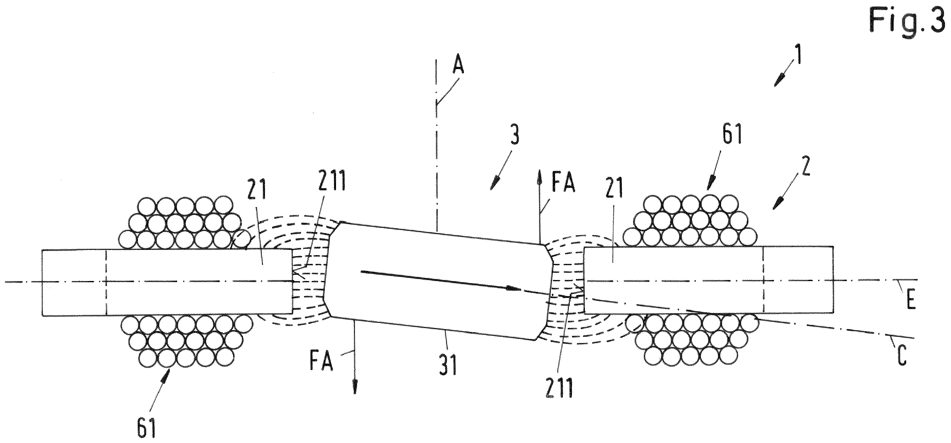

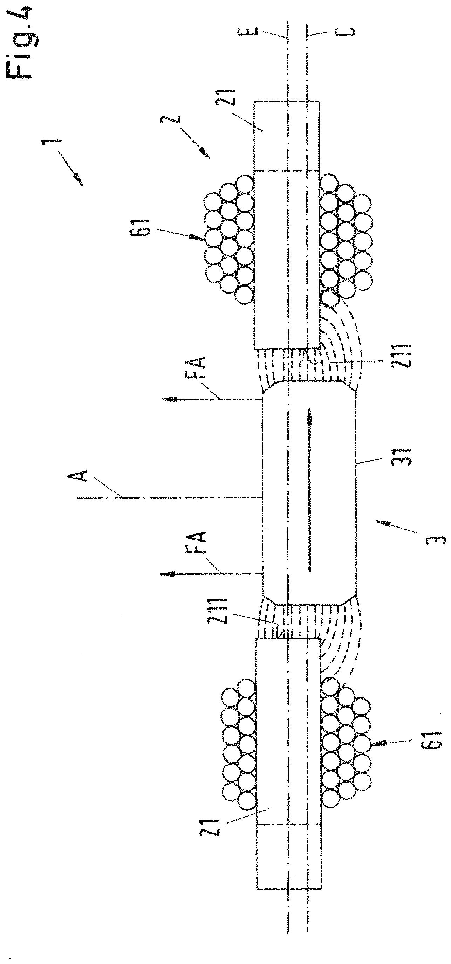

Während des Betriebs des elektromagnetischen Drehantriebs 1 wirkt der magnetisch wirksame Kern 31 des Rotors 3 mit den Statorpolen 21 des Stators 2 nach dem eingangs beschriebenen Prinzip des lagerlosen Motors zusammen, bei welchem der Rotor 3 berührungslos magnetisch antreibbar und berührungslos magnetisch bezüglich des Stators 2 lagerbar ist. Dazu ist der Stator 2 als Lager- und Antriebsstator ausgestaltet, mit welchem der Rotor 3 im Betriebszustand berührungslos magnetisch um die Solldrehachse antreibbar - also in Rotation versetzbar- und bezüglich des Stators 2 berührungslos magnetisch lagerbar ist. Dabei sind drei Freiheitsgrade des Rotors 3, nämlich seine Position in der radialen Ebene E und seine Rotation, aktiv regelbar. Bezüglich seiner axialen Auslenkung aus der radialen Ebene E in axialer Richtung A ist der magnetisch wirksame Kern 31 des Rotors 3 passiv magnetisch, das heisst nicht ansteuerbar, durch Reluktanzkräfte stabilisiert. Auch bezüglich der verbleibenden zwei Freiheitsgrade, nämlich Verkippungen bezüglich der zur Solldrehachse senkrechten radialen Ebene E ist der magnetisch wirksame Kern 31 des Rotors 3 ebenfalls passiv magnetisch stabilisiert, was weiter hinten noch anhand der Fig. 3 und Fig. 4 erläutert wird. Der Rotor 3 ist also durch das Zusammenwirken des magnetisch wirksamen Kerns 31 mit den Statorpolen 21 in axialer Richtung A sowie gegen Verkippungen (insgesamt drei Freiheitsgrade) passiv magnetisch gelagert oder passiv magnetisch stabilisiert und in der radialen Ebene (zwei Freiheitsgrade) aktiv magnetisch gelagert.

Wie dies allgemein üblich ist bezeichnet auch im Rahmen dieser Anmeldung eine aktive magnetische Lagerung eine solche, die aktiv steuer- bzw. regelbar ist, beispielsweise über die mit den konzentrierten Wicklungen 61 generierten elektromagnetischen Drehfelder. Eine passive magnetische Lagerung oder eine passive magnetische Stabilisierung bezeichnet eine solche, die nicht ansteuerbar bzw. regelbar ist. Die passive magnetische Lagerung oder Stabilisierung basiert beispielsweise auf Reluktanzkräften, welche den Rotor 3 bei einer Auslenkung aus seiner Soll-Position, also z. B. bei einer Verschiebung oder Auslenkung in axialer Richtung A oder bei einer Verkippung, wieder in seine Soll-Position bringen.

Mit einer Radiallagerung oder einer radialen Lagerung wird eine Lagerung des Rotors 3 bezeichnet, mit welcher die radiale Position des Rotors 3 stabilisiert werden kann, also eine Lagerung, welche den Rotor 3 in der radialen Ebene und damit bezüglich seiner radialen Position lagert.

Mit einer Axiallagerung oder einer axialen Lagerung bzw. mit einer Axialstabilisierung oder einer axialen Stabilisierung wird eine Lagerung bzw. eine Stabilisierung des Rotors 3 bezeichnet, mit welcher zum einen die Position des Rotors 3 bezüglich der axialen Richtung A stabilisiert wird, und mit welcher zum anderen der Rotor 3 gegen Verkippungen stabilisiert ist. Solche Verkippungen stellen zwei Freiheitsgrade dar und bezeichnen Auslenkungen, bei denen die momentane Drehachse des Rotors 3 nicht mehr genau in die axiale Richtung A zeigt, sondern einen von Null verschiedenen Winkel mit der Solldrehachse einschliesst. Bei einer Verkippung liegt also die magnetische Mittelebene C nicht mehr in oder parallel zur radialen Ebene E, sondern die magnetische Mittelebene C schliesst einen von Null verschiedenen Winkel mit der radialen Ebene E ein.

Beim lagerlosen Motor werden im Unterschied zu klassischen Magnetlagern die magnetische Lagerung und der Antrieb des Motors über elektromagnetische Drehfelder realisiert. Typischerweise wird in dem lagerlosen Motor die magnetische Antriebs- und Lagerfunktion durch die Überlagerung zweier magnetischer Drehfelder generiert, die üblicherweise als Antriebs- und Steuerfeld bezeichnet werden. Diese beiden mit den Wicklungen des Stators 2 erzeugten Drehfelder haben in der Regel eine Polpaarzahl, die sich um eins unterscheidet. Hat also beispielsweise das Antriebsfeld die Polpaarzahl p, so hat das Steuerfeld die Polpaarzahl p+1 oder p-1. Dabei werden mit dem Antriebsfeld auf den magnetisch wirksamen Kern 31 in der radialen Ebene wirkende Tangentialkräfte erzeugt, die ein Drehmoment bewirken, was die Rotation um die axiale Richtung A bewirkt. Durch die Überlagerung des Antriebsfelds und des Steuerfelds lässt sich zusätzlich eine beliebig einstellbare Querkraft auf den magnetisch wirksame Kern 31 in der radialen Ebene erzeugen, mit welcher die Position des magnetisch wirksamen Kerns 31 in der radialen Ebene regelbar ist. Es ist also nicht möglich, den elektromagnetischen Fluss, der von den konzentrierten Wicklungen 61 generiert wird, aufzuteilen in einen (elektro-) magnetischen Fluss, der nur für den Antrieb der Rotation sorgt und einen (elektro-) magnetischen Fluss, der nur die magnetische Lagerung realisiert.

Zur Generierung des Antriebs- und des Steuerfelds ist es einerseits möglich, zwei unterschiedliche Wicklungssysteme zu verwenden, nämlich eines zur Erzeugung des Antriebsfelds und eines zur Erzeugung des Steuerfelds. Die Spulen zur Erzeugung des Antriebsfelds werden dann üblicherweise als Antriebsspulen bezeichnet und die Spulen zur Erzeugung des Steuerfelds als Steuerspulen. Der Strom, der in diese Spulen eingeprägt wird, wird dann als Antriebsstrom bzw. als Steuerstrom bezeichnet. Andererseits ist es aber auch möglich, die Antriebs- und Lagerfunktion mit nur einem einzigen Wicklungssystem - wie in dem hier beschriebenen Ausführungsbeispiel - zu generieren, sodass es also keine Unterscheidung zwischen Antriebs- und Steuerspulen gibt. Dies kann so realisiert werden, dass die von einer Kontrolleinrichtung jeweils ermittelten Werte für den Antriebs- und den Steuerstrom rechnerisch -also z. B. mit Hilfe von Software - addiert bzw. überlagert werden und der sich daraus ergebende Gesamtstrom in die jeweilige konzentrierte Wicklung 61 eingeprägt wird. In diesem Fall ist es natürlich nicht mehr möglich, zwischen Steuer- und Antriebsspulen zu unterscheiden. In dem hier beschriebenen Ausführungsbeispiel ist die letztgenannte Variante realisiert, das heisst, im Stator 2 gibt es keine Unterscheidung zwischen Antriebs- und Steuerspulen, sondern es gibt jeweils nur ein Wicklungssystem, in dessen sechs konzentrierten Wicklungen 61 die rechnerisch ermittelte Summe aus dem Antriebs- und dem Steuerstrom eingeprägt wird. Es ist aber natürlich auch möglich, den erfindungsgemässen elektromagnetischen Drehantrieb 1 so auszugestalten, dass im Stator 2 zwei getrennten Wicklungssysteme, nämlich eines mit separaten Antriebsspulen und eines mit separaten Steuerspulen, vorgesehen sind. Dann sind beispielsweise auf jedem Statorpol 21 jeweils zwei konzentrierte Wicklungen vorgesehen, von denen eine als Antriebsspule dient und eine als Steuerspule.

Um insbesondere die passive magnetische Stabilisierung des Rotors 3 zu verbessern, ist erfindungsgemäss der magnetisch wirksame Kern 31 des Rotors 3 so ausgestaltet, dass er eine Rotorhöhe HR (siehe auch Fig. 5) aufweist, die grösser ist als eine Statorpolhöhe HS (Fig. 1). Die Rotorhöhe HR ist dabei gegeben durch die maximale Erstreckung des magnetisch wirksamen Kerns 31 in axialer Richtung A.

Bei dem ersten Ausführungsbeispiel (siehe insbesondere Fig. 5) ist der magnetisch wirksame Kern 31 bezüglich der axialen Richtung A durch eine erste axiale Begrenzungsfläche 311 und durch eine zweite axiale Begrenzungsfläche 312 begrenzt, die beide senkrecht auf der axialen Richtung A stehen und somit parallel zueinander sind. Der senkrechte Abstand zwischen der ersten axialen Begrenzungsfläche 311 und der zweiten axialen Begrenzungsfläche 312 ist bei dieser Ausgestaltung die Rotorhöhe HR.

Die Statorpolhöhe HS ist durch die maximale Erstreckung der Stirnflächen 211 der Statorpole 21 in der axialen Richtung A definiert. Vorzugsweise haben alle Stirnflächen 211 in der axialen Richtung die gleiche Erstreckung, sodass jede Stirnfläche 211 in der axialen Richtung A die gleiche maximale Erstreckung aufweist, nämlich die Statorpolhöhe HS. Ferner ist es bevorzugt, dass jede Stirnfläche 211 so ausgestaltet ist, dass ihre axiale Höhe in Umfangsrichtung gesehen konstant ist. Dann ist die axiale Höhe jeder Stirnfläche 211 gleich der Statorpolhöhe HS.

Durch die erfindungsgemässe Ausgestaltung des magnetisch wirksamen Kerns 31 des Rotors 3 lässt sich die axiale Steifigkeit der magnetischen Lagerung bzw. der magnetischen Stabilisierung des Rotors deutlich verbessern, denn die höhere Ausgestaltung des magnetisch wirksamen Kerns 31 bezüglich der axialen Richtung A führt zu einer Konzentration der magnetischen Flussdichte im Luftspalt zwischen den Stirnflächen 211 der Statorpole 21 und dem magnetisch wirksamen Kern 31 des Rotors 3. Durch diese Konzentration der Flussdichte im Luftspalt resultiert auch ein deutlich stärkerer Gradient der magnetischen Flussdichte beim Übergang von dem (zumindest näherungsweise) homogenen Feld zwischen den Stirnflächen 211 und dem magnetisch wirksamen Kern 31 in den Bereich des Streufelds, dass bezüglich der axialen Richtung A oberhalb bzw. unterhalb der Statorpole 21 herrscht. In den Zeichnungsfiguren, beispielsweise in Fig. 1 bis Fig. 4 sind die Feldlinien des magnetischen Flusses jeweils durch die strichlierten Linien zwischen den Statorpolen 21 und dem magnetisch wirksamen Kern 31 dargestellt, wobei gerade, parallele Feldlinien den Bereich des homogenen Feldes anzeigen und gekrümmte Feldlinien den Bereich der Streufelder.

Da die axiale Steifigkeit der magnetischen Lagerung quadratisch mit der magnetischen Flussdichte und damit überproportional mit der Rotorhöhe HR ansteigt, lässt sich mit der erfindungsgemässen Ausgestaltung eine deutlich Verbesserung der axialen Steifigkeit der magnetischen Lagerung erzielen. Ferner lässt sich mit dieser Ausgestaltung durch die Konzentration des magnetischen Flusses im Luftspalt auch das Drehmoment vergrössern, welches die Rotation des Rotors 3 antreibt.

Bei der hier beschriebenen Ausgestaltung (siehe Fig. 5) hat der magnetisch wirksamen Kern 31 einen zentralen Bereich 32, welcher bezüglich der axialen Richtung A zwischen einem ersten Randbereich 33 und einem zweiten Randbereich 34 angeordnet ist, wobei beide Randbereiche 33, 34 unmittelbar an den zentralen Bereich 32 angrenzen. Der zentrale Bereich 32 weist einen Durchmesser auf, welcher als Rotordurchmesser RZ bezeichnet wird. In axialer Richtung A weist der zentrale Bereich 32 eine zentrale Höhe HZ auf, welche kleiner ist als die Rotorhöhe HR.

Der erste Randbereich 33 bildet die erste axiale Begrenzungsfläche 311 des magnetisch wirksamen Kerns 31, wobei die erste axiale Begrenzungsfläche 311 einen ersten Randdurchmesser R1 aufweist, welches ihr Aussendurchmesser ist. Der zweite Randbereich 34 bildet die zweite axiale Begrenzungsfläche 312 des magnetisch wirksamen Kerns 31, wobei die zweite axiale Begrenzungsfläche 312 einen zweiten Randdurchmesser R2 aufweist, welches ihr Aussendurchmesser ist. Jeder der Randdurchmesser R1 und R2 ist kleiner als der Rotordurchmesser RZ. Dabei können der erste Randdurchmesser R1 und der zweite Randdurchmesser R2 gleich gross sein, so wie dies in Fig. 5 dargestellt ist. In anderen Ausgestaltungen können der erste Randdurchmesser und der zweite Randdurchmesser unterschiedlich gross sein.

Bei der in Fig. 5 dargestellten Ausgestaltung des magnetisch wirksamen Kerns 31 hat der zentrale Bereich 32 in einem Axialschnitt ein rechteckiges Profil, welches die zentrale Höhe HZ als Höhe aufweist und den Rotordurchmesser RZ als Breite.

Besonders bevorzugt ist der zentrale Bereich 32 des magnetisch wirksamen Kerns 31 so ausgestaltet, dass die zentrale Höhe HZ gleich gross ist wie die Statorpolhöhe HS.

Der erste Randbereich 33 und der zweite Randbereich 34 sind jeweils in Form eines Kegelstumpfs ausgestaltet, wobei der Kegelstumpf an seiner Basis jeweils einen Durchmesser aufweist, welcher dem Rotordurchmesser RZ entspricht und an seiner der Basis abgewandten axialen Begrenzungsfläche 311, 312 einen kleineren Durchmesser, welcher dem ersten Randdurchmesser R1 bzw. dem zweiten Randdurchmesser R2 entspricht.

Vorzugsweise - aber nicht notwendigerweise - sind der erste Randbereich 311 und der zweite Randbereich 312 gleichartig ausgestaltet.

Es sind auch solche Ausgestaltungen möglich, bei welchen der erste Randbereich 33 und/oder der zweite Randbereich 34 kreisscheibenförmig mit einem Durchmesser ausgestaltet sind, welcher dem ersten Randdurchmesser R1 bzw. dem zweiten Randdurchmesser R2 entsprechen, sodass dann der erste Randbereich 33 und/oder der zweite Randbereich 34 in einem Axialschnitt ein rechteckiges Profil aufweisen.

Bevorzugt sind jedoch solche Ausgestaltungen des magnetisch wirksamen Kerns 31, bei denen der magnetisch wirksame Kern 31 eine Aussenfläche aufweist, die weder zwischen dem zentralen Bereich 32 und der ersten axialen Begrenzungsfläche 311 noch zwischen dem zentralen Bereich 32 und der zweiten axialen Begrenzungsfläche 312 parallel zur axialen Richtung A verläuft. Es sind also solche Ausgestaltungen bevorzugt, bei welchen sowohl die erste axiale Begrenzungsfläche 311 als auch die zweite axiale Begrenzungsfläche durch Übergänge 35 mit dem zentralen Bereich 32 verbunden sind, die schräg zur axialen Richtung A oder gekrümmt verlaufen.

Durch diese Massnahme wird die Kippsteifigkeit des Rotors 3, das heisst sein Widerstand gegen Verkippungen, bzw. seine Fähigkeit aus einer verkippten Position in die Soll-Position zurückzukehren, deutlich verbessert.

Wie bereits erwähnt sind bei der in Fig. 5 dargestellten Ausgestaltung der erste Randbereich 33 und der zweite Randbereich 34 jeweils in Form eines Kegelstumpfs ausgestaltet. Die Neigung des Kegelstumpfs wird dabei durch einen Kegelstumpfwinkel α beschrieben, welcher durch den spitzen Winkel zwischen dem Übergang 35 und der axialen Richtung A gegeben ist.

In der Praxis haben sich bestimmte Kombinationen der geometrischen Abmessungen als besonders vorteilhaft erwiesen.

Für das Höhenverhältnis aus der Rotorhöhe HR und der zentralen Höhe HZ ist der Bereich von 1.2 bis 1.6 bevorzugt, also 1.2 ≤ HR/HZ ≤ 1.6, wobei der Kegelstumpfwinkel α zwischen 15 Grad und 60 Grad ist, also 15° ≤ α ≤ 60°. Dabei hat es sich als vorteilhaft erwiesen, wenn der Kegelstumpfwinkel α umso grösser ist, je grösser das Höhenverhältnis HR/HZ ist.

Ferner hat es sich als vorteilhaft erwiesen, wenn das Verhältnis aus dem Rotordurchmesser RZ und der Rotorhöhe HR zwischen zwei und drei liegt, also 2 ≤ RZ/HR ≤ 3, wobei dieses Verhältnis vorzugsweise umso kleiner gewählt werden kann, desto grösser der Kegelstumpfwinkel α ist.

Besonders bevorzugt ist das Höhenverhältnis aus der Rotorhöhe HR und der zentralen Höhe HZ im Bereich von 1.3 bis 1.5, also 1.3 ≤ HR/HZ ≤ 1.5, wobei der Kegelstumpfwinkel α zwischen 20 Grad und 30 Grad ist, also 20° ≤ α ≤ 30°. Für das Verhältnis aus dem Rotordurchmesser RZ und der Rotorhöhe HR ist der Bereich von 2.3 bis 2.7 besonders bevorzugt, also 2.3 ≤ RZ/HR ≤ 2.7.

Speziell bevorzugt ist das Höhenverhältnis aus der Rotorhöhe HR und der zentralen Höhe HZ etwa 1.46, also HR/HZ = 1.46, wobei der Kegelstumpfwinkel α etwa 22.5 Grad ist, also α = 22.5°.

In den Fig. 3 und Fig. 4 ist die axiale Steifigkeit der passiven magnetischen Lagerung des Rotors 3 gegen Verkippungen bzw. gegen Verschiebungen des Rotors 3 in axialer Richtung A veranschaulicht. Dabei sind die Feldlinien des magnetischen Flusses zwischen dem magnetisch wirksamen Kern des Rotors 3 und den Statorpolen 21 wiederum durch die strichlierten Linien ohne Bezugszeichen dargestellt.

Fig. 3 zeigt den magnetisch wirksamen Kern des Rotors in einer verkippten Stellung, bei welcher die magnetische Mittelebene C mit der axialen Richtung A einen von Null verschiedenen Winkel einschliesst. In dieser Position wirkt in dem Bereich des magnetisch wirksamen Kerns 31, welcher der darstellungsgemäss linken Stirnfläche 211 gegenüberliegt, in axialer Richtung A eine Kraftkomponente FA, die darstellungsgemäss nach unten gerichtet ist, während in dem Bereich des magnetisch wirksamen Kerns 31 welcher der darstellungsgemäss rechten Stirnfläche 211 gegenüberliegt in axialer Richtung A eine Kraftkomponente FA wirkt, die darstellungsgemäss nach oben gerichtet ist. Die beiden Kraftkomponenten FA üben somit ein Drehmoment auf den magnetisch wirksamen Kern 31 aus, welches diesen zurück in seine Soll-Position, also in eine unverkippte Position bringt, in welcher die magnetische Mittelebene C senkrecht auf der axialen Richtung A steht.

Fig. 4 zeigt den magnetisch wirksamen Kern 31 des Rotors 3 in einer bezüglich der axialen Richtung A verschobenen bzw. ausgelenkten Position. In dieser steht die magnetische Mittelebene C zwar noch senkrecht auf der axialen Richtung A, ist aber bezüglich der radialen Ebene E parallel nach unten (darstellungsgemäss) verschoben. In dieser Position wirkt in dem Bereich des magnetisch wirksamen Kerns 31, welcher der darstellungsgemäss linken Stirnfläche 211 gegenüberliegt, in axialer Richtung A eine Kraftkomponente FA, die darstellungsgemäss nach oben gerichtet ist, und in dem Bereich des magnetisch wirksamen Kerns 31, welcher der darstellungsgemäss rechten Stirnfläche 211 gegenüberliegt wirkt in axialer Richtung eine Kraftkomponente FA, die darstellungsgemäss ebenfalls nach oben gerichtet ist. Diese beiden Kraftkomponenten FA üben somit ein Kraft auf den magnetisch wirksamen Kern 31 aus, welche diesen zurück in seine Soll-Position bringt, in welcher die magnetische Mittelebene C in der radialen Ebene E liegt.

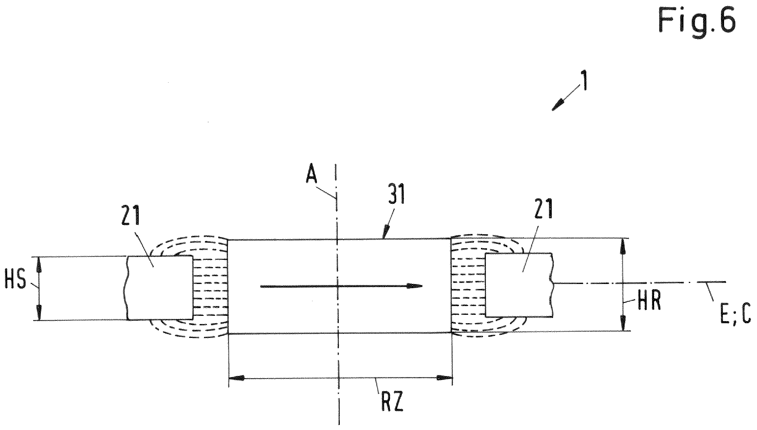

Im Folgenden werden anhand der Fig. 6 bis Fig. 9 verschiedene Varianten für die Ausgestaltung des Rotors 3 bzw. des magnetisch wirksamen Kerns 31 des Rotors 3 erläutert. Dabei wird nur auf die Unterschiede bezüglich der bisherigen Erläuterungen eingegangen. Ansonsten gelten die bisherigen Erläuterungen in gleicher oder in sinngemäss gleicher Weise auch für diese Varianten. Ferner ist es möglich, die anhand der Varianten beschriebenen Massnahmen auch miteinander zu kombinieren.

Fig. 6 veranschaulicht eine erste Variante für den magnetisch wirksamen Kern 31 des Rotors 3. Bei dieser ersten Variante ist der magnetisch wirksame Kern 31 gesamthaft mit einer Höhe in axialer Richtung A ausgestaltet, welche gleich der Rotorhöhe HR ist. Bei dieser Ausgestaltung hat der magnetisch wirksame Kern 31 keine ausgeprägten Randbereiche, die von einem zentralen Bereich unterscheidbar wären. Der magnetisch wirksame Kern ist als Kreisscheibe ausgestaltet, bzw. als Scheibe eines Kreiszylinders, wobei diese Scheibe den Durchmesser hat, welcher der Rotordurchmesser RZ ist, und eine Höhe in axialer Richtung, welches die Rotorhöhe HR ist. Der magnetisch wirksame Kern 31 kann natürlich auch als eine Ringscheibe ausgestaltet sein.

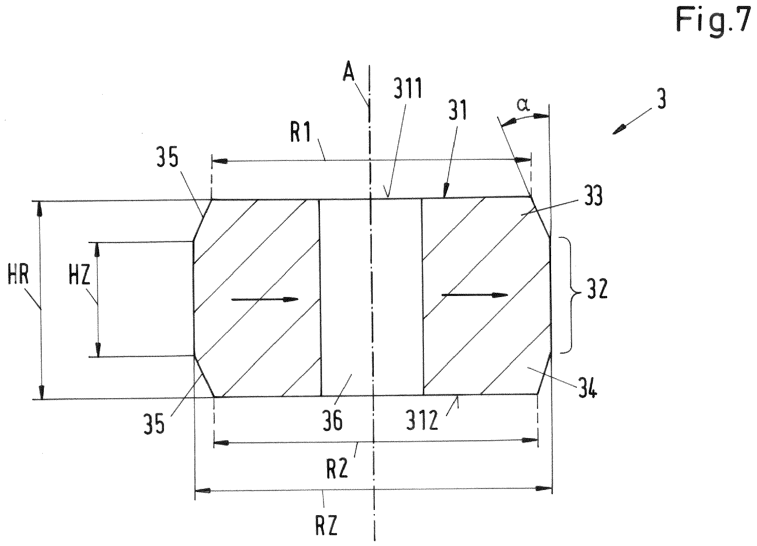

Fig. 7 zeigt eine zweite Variante für den magnetisch wirksamen Kern 31 in einem Schnitt in axialer Richtung. Die zweite Variante entspricht weitgehend der in Fig. 5 dargestellten Ausführungsform, weist jedoch eine zentrale Bohrung 36 auf, die sich in axialer Richtung A durch den gesamten magnetisch wirksamen Kern 31 von der ersten axialen Begrenzungsfläche 311 bis zu der zweiten axialen Begrenzungsfläche 312 erstreckt.

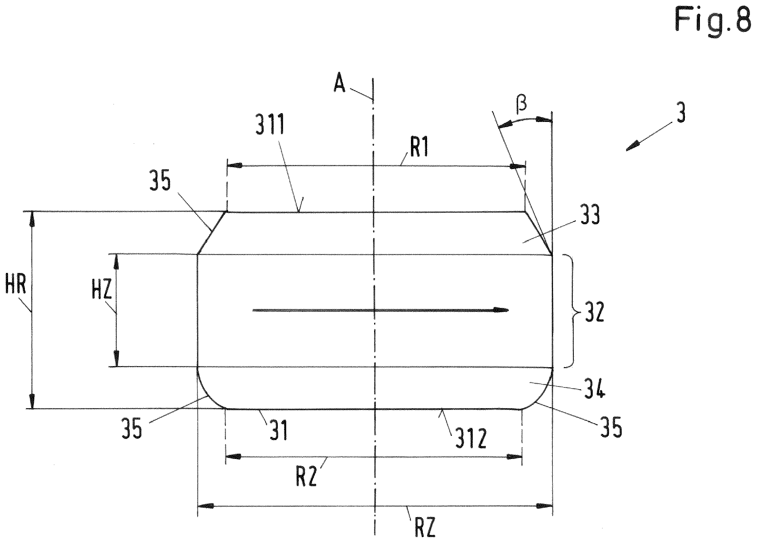

Fig. 8 zeigt eine dritte Variante für den magnetisch wirksamen Kern 31. Die dritte Variante entspricht weitgehend der in Fig. 5 dargestellten Ausführungsform, unterscheidet sich jedoch durch die Ausgestaltung des ersten Randbereichs 33 und des zweiten Randbereichs 34. Bei der dritten Variante sind beide Randbereiche 33, 34 jeweils in Form einer Kugelscheibe oder in Form einer Paraboloidscheibe ausgestaltet, das heisst, die Übergänge 35 sind hierbei jeweils gekrümmt ausgestaltet, beispielsweise als Teil einer Kugelschale oder als Teil eines Paraboloids. Die Neigung der Übergänge 35 kann beispielsweise durch einen Neigungswinkel β beschrieben werden, der in sinngemäss gleicher Weise wie der Kegelstumpfwinkel α festgelegt werden kann. So ist der Neigungswinkel β beispielsweise der spitze Winkel, welchen eine Tangente an den Übergang 35 mit der axialen Richtung A einschlicht. Für den Neigungswinkel β gelten sinngemäss die gleichen Erläuterungen wie für den Kegelstumpfwinkel α.

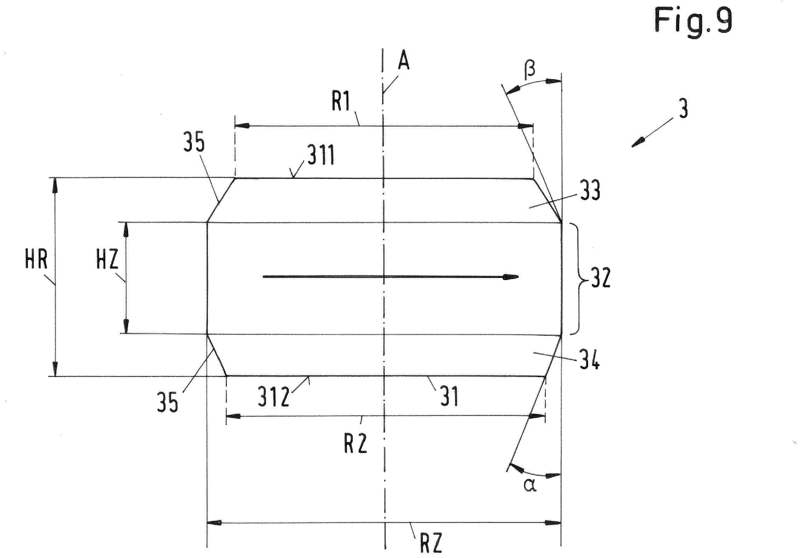

Fig. 9 zeigt eine vierte Variante für den magnetisch wirksamen Kern 31. Die vierte Variante entspricht weitgehend der in Fig. 5 dargestellten Ausführungsform, unterscheidet sich jedoch durch die Ausgestaltung des ersten Randbereichs 33. Bei der vierten Variante ist nur der zweite Randbereich 34 in Form eines Kegelstumpfs ausgestaltet, während der erste Randbereich in Form einer Kugelscheibe oder in Form einer Paraboloidscheibe ausgestaltet ist.

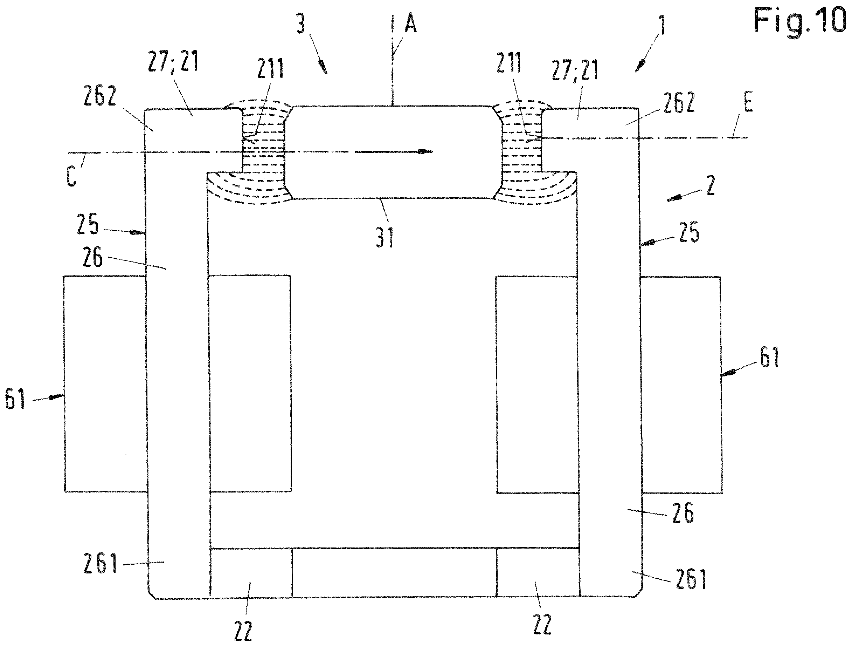

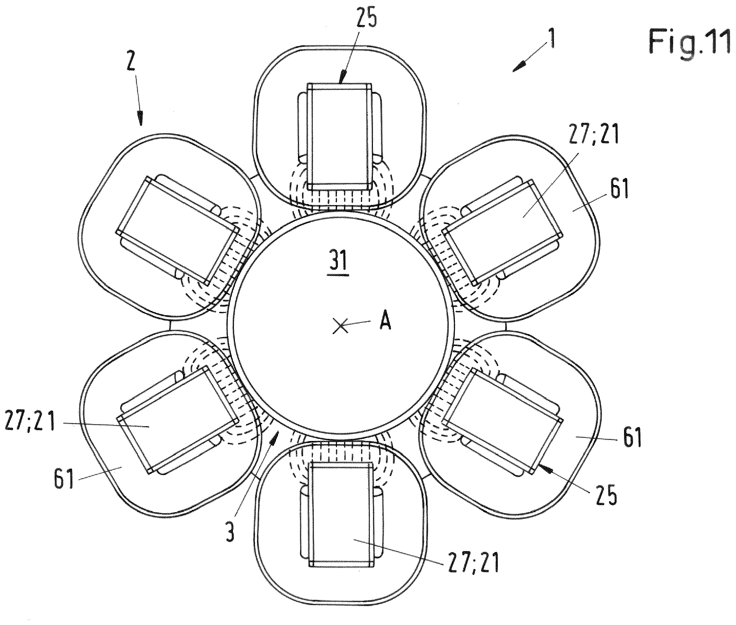

Fig. 10 zeigt ein zweites Ausführungsbeispiel eines erfindungsgemässen elektromagnetischen Drehantriebs in einem Schnitt in axialer Richtung A. Zum besseren Verständnis zeigt Fig. 11 noch eine Aufsicht auf das zweite Ausführungsbeispiel aus der axialen Richtung A.

Im Folgenden wird nur auf die Unterschiede zu dem ersten Ausführungsbeispiel eingegangen. Gleiche Teile oder von der Funktion her gleichwertige Teile des zweiten Ausführungsbeispiels sind mit den gleichen Bezugszeichen bezeichnet wie bei dem ersten Ausführungsbeispiel bzw. seinen Varianten. Insbesondere haben die Bezugszeichen die gleiche Bedeutung wie sie bereits im Zusammenhang mit dem ersten Ausführungsbeispielen erläutert sind. Es versteht sich, dass alle vorangehenden Erläuterungen des ersten Ausführungsbeispiels und seiner Varianten in gleicher Weise oder in sinngemäss gleicher Weise auch für das zweite Ausführungsbeispiel gelten.

Bei dem zweiten Ausführungsbeispiel ist der elektromagnetische Drehantrieb 1 als Tempelmotor ausgestaltet Der elektromagnetische Drehantrieb 1 umfasst den Stator 2, wobei der Stator 2 eine Mehrzahl von Spulenkernen 25 aufweist, von denen jeder einen stabförmigen Längsschenkel 26 umfasst, welcher sich von einem ersten Ende 261 in axialer Richtung A bis zu einem zweiten Ende 262 erstreckt, sowie einen Querschenkel 27, welcher an dem zweiten Ende 262 des Längsschenkels 26 angeordnet ist, Jeder Querschenkel 27 erstreckt sich in der radialen Richtung auf den Rotor 3 zu. Jeder Spulenkern 25 hat somit eine L-förmige Ausgestaltung, wobei die Längsschenkel 26 jeweils den sich in axialer Richtung A erstreckenden langen Schenkel des L bilden, und die sich senkrecht zu den Längsschenkeln 26 in radialer Richtung auf den Rotor 3 hin gerichtet erstreckenden Querschenkel 27 jeweils den kurzen Schenkel des L bilden.

Jeder Querschenkel 27 bildet einen der Statorpole 21. Im Unterschied zu dem ersten Ausführungsbeispiel, welches als Radialmotor ausgestaltet ist, werden die konzentrierten Wicklungen 61 nicht von den Statorpolen 21 getragen, sondern an jedem Längsschenkel 26 ist mindestens eine der konzentrierten Wicklungen 61 angeordnet, welche den jeweiligen Längsschenkel 26 umgibt.

Wenn sich der magnetisch wirksame Kern 31 des Rotors 3 während des Betriebs in seiner Soll-Position befindet, so ist der magnetisch wirksame Kern 31 zwischen den Statorpolen 21, welche durch die Querschenkel 27 gebildet werden, zentriert, sodass die Statorpole 21 in der magnetischen Mittelebene C bzw. in der radialen Ebene E (diese zwei Ebenen sind in diesem Fall gleich) angeordnet sind. Die konzentrierten Wicklungen 61 sind darstellungsgemäss unterhalb der radialen Ebene E angeordnet und so ausgerichtet, dass ihre Spulenachsen in axialer Richtung A verlaufen.

Alle ersten Enden 261 der Längsschenkel 26 - gemäss der Darstellung in Fig. 10 sind dies die unteren Enden - sind durch den Rückschluss 22 miteinander verbunden. Der Rückschluss 22 ist vorzugsweise ringförmig ausgestaltet. Dabei sind solche Ausgestaltungen möglich (siehe Fig. 10), bei welchen sich der Rückschluss 22 radial innenliegend entlang aller ersten Enden 261 der Längsschenkel erstreckt. Es ist aber auch möglich, dass der Rückschluss 22 entlang seines Umfangs mehrere Ausnehmungen aufweist, von denen jede eines der ersten Enden 262 aufnimmt.

Durch die Erfindung wird ferner eine Zentrifugalpumpe 100 zum Fördern eines Fluids vorgeschlagen, die dadurch gekennzeichnet ist, dass die Zentrifugalpumpe 100 einen elektromagnetischen Drehantrieb 1 umfasst, der erfindungsgemäss ausgestaltet ist, wobei der Rotor 3 des elektromagnetischen Drehantriebs 1 als Rotor 3 der Zentrifugalpumpe 100 ausgestaltet ist.

Fig. 12 zeigt ein Ausführungsbeispiel einer erfindungsgemässen Zentrifugalpumpe, die gesamthaft mit dem Bezugszeichen 100 bezeichnet ist, in einem Schnitt in axialer Richtung A.

Bei diesem Ausführungsbeispiel der Zentrifugalpumpe 100 ist der elektromagnetische Drehantrieb 1 als Tempelmotor, also gemäss dem zweiten Ausführungsbeispiel (Fig. 10, Fig. 11) ausgestaltet.

Die Zentrifugalpumpe 100 umfasst eine Pumpeneinheit 50 mit einem Pumpengehäuse 51, das einen Einlass 52 und einen Auslass 53 für das zu fördernde Fluid umfasst, wobei der Rotor 3 im Pumpengehäuse 51 angeordnet ist, und eine Mehrzahl von Flügeln 54 zum Fördern des Fluids umfasst. Die Pumpeneinheit 50 ist derart ausgestaltet, dass die Pumpeneinheit 50 so in den Stator 2 einsetzbar ist, dass der magnetisch wirksame Kern des Rotors 31 von den Statorpolen 21 umgeben wird.

Ein vorteilhafter Aspekt ist es, dass der Rotor 3 als Integralrotor ausgestaltet ist, weil er sowohl der Rotor 3 des elektromagnetischen Drehantriebs 1 ist, als auch der Rotor 3 der Zentrifugalpumpe 100, mit welchem das Fluid gefördert wird. Insgesamt erfüllt der Rotor 3 somit drei Funktionen in einem: Er ist der Rotor 3 des elektromagnetischen Antriebs 1, er ist der Rotor 3 der magnetischen Lagerung, und er ist das Laufrad, mit welchem auf das Fluid bzw. die Fluide eingewirkt wird. Diese Ausgestaltung als Integralrotor bietet den Vorteil einer sehr kompakten und platzsparenden Ausgestaltung.

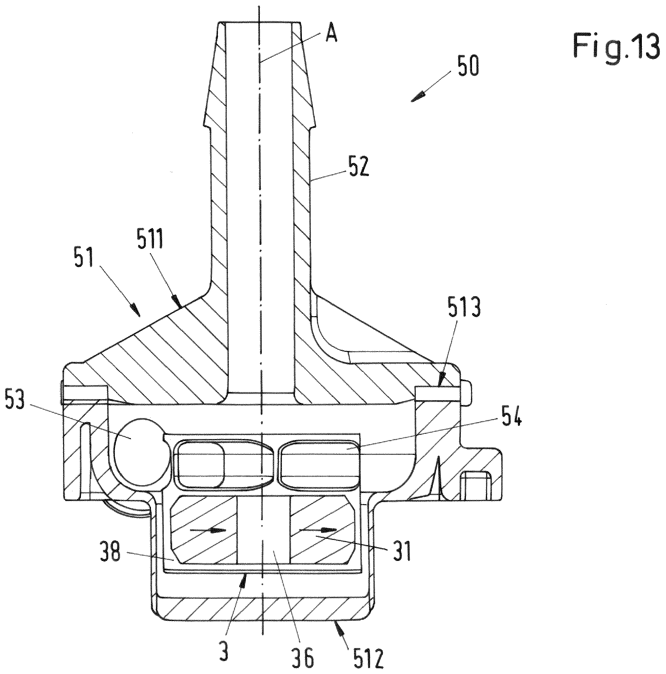

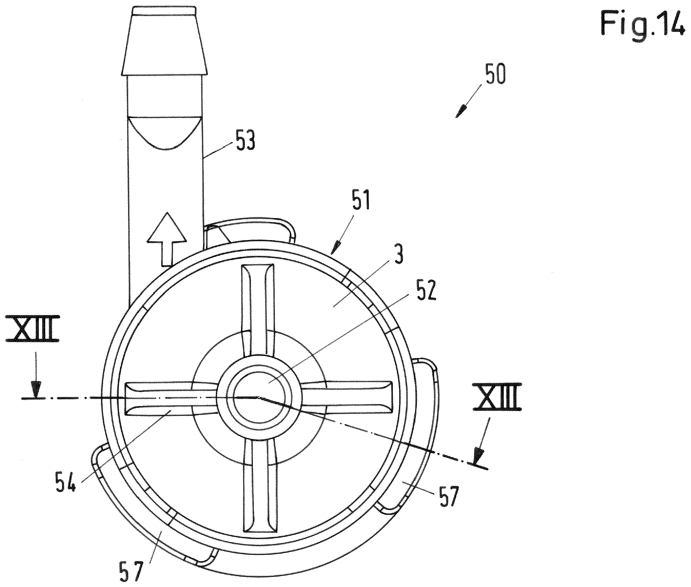

Zum besseren Verständnis zeigt Fig. 13 noch eine etwas detailliertere Darstellung der Pumpeneinheit 50 der Zentrifugalpumpe 100 in einem Schnitt entlang der Schnittlinie XIII-XIII in Fig. 14. Fig. 14 zeigt eine Aufsicht auf die Pumpeneinheit 50 aus der axialen Richtung A.

Das Pumpengehäuse 51 der Pumpeneinheit umfasst ein Basisteil 512 und einen Deckel 511, welche dichtend miteinander verbunden sind, wobei der Auslass 53 des Pumpengehäuses 51 vollständig im Basisteil 512 angeordnet ist. Der Deckel 511 umfasst den Einlass 52, welcher sich in axialer Richtung A erstreckt, sodass das Fluid den Rotor 3 aus der axialen Richtung A anströmt.

Ein wesentlicher Aspekt ist hierbei auch, dass der Auslass 53 vollständig im Basisteil 512 angeordnet ist, sodass der Auslass 53 keine Trennfugen, Schweissnähte oder ähnliche Verbindungsstellen aufweist.

Zum dichtenden Verbinden des Deckels 511 und des Basisteils 512 sind alle an sich bekannten Methoden geeignet. So können das Basisteil 512 und der Deckel 511 beispielsweise durch eine Verschraubung oder durch eine Klickverbindung oder durch eine Einrastverbindung, durch Verkleben oder durch verschiedene Arten von Verschweissungen, beispielsweise durch Infrarotschweissen, miteinander verbunden werden. Je nach Art der Verbindung kann es vorteilhaft sein, zwischen dem Basisteil 512 und dem Deckel 511 ein Dichtungselement 513, beispielsweise einen O-Ring vorzusehen.

Der Rotor 3 umfasst die Mehrzahl von Flügeln 54 zum Fördern des Fluids. Bei dem hier beschriebenen Ausführungsbeispiel sind insgesamt vier Flügel 54 vorgesehen, wobei diese Anzahl beispielhaften Charakter hat. Der Rotor 3 umfasst ferner eine Ummantelung 38, mit welcher der magnetisch wirksame Kern 31 des Rotors 3 umschlossen und vorzugsweise hermetisch eingekapselt ist, sodass der magnetisch wirksame Kern 31 des Rotors 3 nicht in Kontakt mit dem zu fördernden Fluid kommt. Alle Flügel 54 sind auf der Ummantelung 38 angeordnet und bezüglich der Umfangsrichtung des Rotors 3 äquidistant angeordnet. Jeder Flügel 54 erstreckt sich in radialer Richtung nach aussen und ist drehfest mit dem Ummantelung 38 verbunden. Die Flügel 54 können separate Komponenten sein, die dann auf der Ummantelung 38 fixiert werden. Es ist natürlich auch möglich, dass alle Flügel 54 integraler Bestandteil der Ummantelung 38 sind, dass also die Ummantelung 38 mit allen Flügeln 54 einstückig ausgestaltet ist. Der Rotor 3 mit den Flügeln 54 bildet das Flügelrad bzw. das Laufrad der Zentrifugalpumpe 100, mit welchem auf das Fluid oder die Fluide eingewirkt wird.

Vorzugsweise umfasst der Rotor 3 die zentrale Bohrung 36, welche sich in axialer Richtung A vollständig durch den Rotor 3 hindurch erstreckt. Durch diese zentrale Bohrung 36 lässt sich ein zumindest teilweiser Axialschubausgleich gewährleisten, sodass die passive magnetische axiale Lagerung des Rotors 3 entlastet wird.

Je nach Anwendungsfall, beispielsweise, wenn die Zentrifugalpumpe als Blutpumpe eingesetzt wird, ist es bevorzugt, wenn das Pumpengehäuse 51 der Pumpeneinheit 50 sowie die Ummantelung 38 und die Flügel 54 aus einem oder mehreren Kunststoffen hergestellt wird. Geeignete Kunststoffe sind: Polyethylene (PE), Low Density Polyethylene (LDPE), Ultra Low Density Polyethylene (ULDPE), Ethylene Vinyl Acetate (EVA), Polyethylene Terephthalate (PET), Polyvinylchlorid (PVC), Polypropylene (PP), Polyurethan (PU), Polyvinylidene Fluoride (PVDF), Acrylonitrile Butadiene Styrene (ABS), Polyacryl, Polycarbonate (PC), Polyetheretherketon (PEEK) oder Silicone. Für viele Anwendungen sind auch die unter dem Markennamen Teflon bekannten Materialien Polytetrafluoroethylene (PTFE) und und Perfluoralkoxy-Polymere (PFA) als Kunststoff geeignet.

Vorzugsweise ist die Pumpeneinheit 50 zum lösbaren Verbinden mit dem Stator 2 der Zentrifugalpumpe 100 ausgestaltet ist. Hierzu können an dem Pumpengehäuse 51 beispielsweise mehrere Laschen 57 vorgesehen sein, die mit dem Stator 2 in Form einer Bajonettverbindung zusammenwirken können.

In einer besonders bevorzugten Ausführungsform ist die Pumpeneinheit 50 als Einmalvorrichtung für den Einmalgebrauch ausgestaltet, die in den als wiederverwendbare Vorrichtung ausgestalteten Stator 2 einsetzbar ist. Die Zentrifugalpumpe 100 setzt sich dann aus der Pumpeneinheit 50 zusammen, die als Einmalvorrichtung für den Einmalgebrauch ausgestaltet ist, sowie dem Stator 2, der als wiederverwendbare Vorrichtung ausgestaltet ist, die für den Mehrfachgebrauch ausgebildet ist. Der Stator 2 umfasst dabei typischerweise auch die Kontroll-, Regel- und Versorgungseinheiten des elektromagnetischen Drehantriebs 1.

Mit dem Begriff "Einmalvorrichtung" und anderen Zusammensetzungen mit dem Bestandteil "Einmal", sind dabei solche Komponenten bzw. Teile gemeint, die für den Einmalgebrauch ausgestaltet sind, die also bestimmungsgemäss nur ein einziges Mal benutzt werden können und dann entsorgt werden. Für eine neue Anwendung muss dann ein neues, bisher unbenutztes Einmalteil eingesetzt werden. Bei der Konzipierung bzw. der Ausgestaltung der Einmalvorrichtung ist es daher ein wesentlicher Aspekt, dass die Einmalvorrichtung in möglichst einfacher Weise mit der wiederverwendbaren Vorrichtung zu der Zentrifugalpumpe zusammenfügbar ist. Die Einmalvorrichtung soll also in sehr einfacher Weise ersetzt werden können, ohne dass dafür ein hoher Montageaufwand notwendig ist. Besonders bevorzugt soll die Einmalvorrichtung ohne die Verwendung von Werkzeugen mit der wiederverwendbaren Vorrichtung zusammenfügbar und von dieser trennbar sein. Die Pumpeneinheit 50 kann als eine solche Einmalvorrichtung ausgestaltet werden.

Die Zentrifugalpumpe 100 kann beispielsweise in der Medizinaltechnik als Blutpumpe eingesetzt werden oder in der pharmazeutischen Industrie oder in der biotechnologischen Industrie Verwendung finden. Speziell eignet sich die Zentrifugalpumpe 100 für solche Anwendungen, bei denen ein sehr hohes Mass an Reinheit oder Sterilität derjenigen Komponenten wesentlich ist, die mit den zu mischenden Substanzen in Kontakt kommen.

Es versteht sich, dass die erfindungsgemässe Zentrifugalpumpe 100 zum Fördern von Fluiden auch mit einem elektromagnetischen Drehantrieb 1 ausgestaltet sein kann, welcher gemäss dem ersten Ausführungsbeispiel (Fig. 1, Fig. 2) ausgestaltet ist, also als Radialmotor, bei welchem die Wicklungen 61 auf den Statorpolen 21 in der radialen Ebene E angeordnet sind.

## Patentansprüche

1. Elektromagnetischer Drehantrieb, der als Innenläufer ausgestaltet ist, mit einem Rotor (3), der einen ring- oder scheibenförmigen magnetisch wirksamen Kern (31) umfasst, welcher von einem radial aussenliegend angeordneten Stator (2) umgeben ist, wobei der Stator (2) eine Mehrzahl von Statorpolen (21) aufweist, die um den magnetisch wirksamen Kern (31) herum angeordnet sind, und von denen jeder jeweils durch eine dem magnetisch wirksamen Kern (31) des Rotors (3) zugewandte Stirnfläche (211) begrenzt wird, wobei der Stator (2) als Lager- und Antriebsstator ausgestaltet ist, mit welchem der Rotor (3) im Betriebszustand berührungslos magnetisch um eine Solldrehachse antreibbar ist, die eine axiale Richtung (A) definiert, und mit welchem der Rotor (3) berührungslos magnetisch bezüglich des Stators (2) lagerbar ist, wobei der Rotor (3) in einer zur axialen Richtung (A) senkrechten radialen Ebene (E) aktiv magnetisch gelagert ist, und in axialer Richtung (A) sowie gegen Verkippungen passiv magnetisch stabilisiert ist, **dadurch gekennzeichnet, dass** der magnetisch wirksame Kern (31) des Rotors (3) eine Rotorhöhe (HR) aufweist, welche die maximale Erstreckung des magnetisch wirksamen Kerns (31) in axialer Richtung (A) ist, wobei die Rotorhöhe (HR) grösser ist als eine Statorpolhöhe (HS), welche durch die maximale Erstreckung der Stirnflächen (211) der Statorpole (21) in axialer Richtung (A) festgelegt ist.

2. Drehantrieb nach Anspruch 1, wobei der magnetisch wirksame Kern (31) einen zentralen Bereich (32) umfasst, welcher bezüglich der axialen Richtung (A) zwischen einem ersten Randbereich (33) und einem zweiten Randbereich (34) angeordnet ist, und welcher einen Rotordurchmesser (RZ) aufweist, wobei der erste Randbereich (33) eine erste axiale Begrenzungsfläche (311) des magnetisch wirksamen Kerns (31) bildet, die einen ersten Randdurchmesser (R1) aufweist, wobei der zweite Randbereich (34) eine zweite axiale Begrenzungsfläche (312) des magnetisch wirksamen Kerns bildet (31), die einen zweiten Randdurchmesser (R2) aufweist, und wobei jeder Randdurchmesser (R1, R2) kleiner ist als der Rotordurchmesser (RZ).

3. Drehantrieb nach Anspruch 2, wobei der zentrale Bereich (32) eine zentrale Höhe (HZ) aufweist, welches die Erstreckung des zentralen Bereichs (32) in axialer Richtung (A) ist, wobei die zentrale Höhe (HZ) gleich gross ist wie die Statorpolhöhe (HS).

4. Drehantrieb nach einem der Ansprüche 2-3, wobei der magnetisch wirksame Kern (31) eine Aussenfläche aufweist, die weder zwischen dem zentralen Bereich (32) und der ersten axialen Begrenzungsfläche (311) noch zwischen dem zentralen Bereich (32) und der zweiten axialen Begrenzungsfläche (312) parallel zur axialen Richtung (A) verläuft.

5. Drehantrieb nach einem der Ansprüche 2-4, wobei mindestens einer des ersten und des zweiten Randbereichs (33, 34) in Form eines Kegelstumpfs oder in Form einer Kugelscheibe oder in Form einer Paraboloidscheibe ausgestaltet ist.

6. Drehantrieb nach einem der Ansprüche 2-5, wobei der erste Randbereich (33) und der zweite Randbereich (34) gleichartig ausgestaltet sind.

7. Drehantrieb nach einem der vorangehenden Ansprüche, wobei jeder Statorpol (21) mindestens eine konzentrierte Wicklung (61) trägt, sodass jede konzentrierte Wicklung (61) in der radialen Ebene (E) angeordnet ist.

8. Drehantrieb nach einem der Ansprüche 1-6, wobei der Drehantrieb als Tempelmotor ausgestaltet ist, und der Stator (2) eine Mehrzahl von Spulenkernen (25) aufweist, von denen jeder einen stabförmigen Längsschenkel (26) umfasst, welcher sich von einem ersten Ende (261) in axialer Richtung (A) bis zu einem zweiten Ende (262) erstreckt, sowie einen Querschenkel (27), welcher an dem zweiten Ende (262) des Längsschenkels (26) und in der radialen Ebene (E) angeordnet ist, und welcher sich in einer radialen Richtung erstreckt, die senkrecht zu der axialen Richtung (A) ist, wobei jeder Querschenkel (27) einen der Statorpole (21) bildet, und wobei an jedem Längsschenkel (26) mindestens eine konzentrierte Wicklung (61) angeordnet ist, welche den jeweiligen Längsschenkel (26) umgibt.

9. Zentrifugalpumpe zum Fördern eines Fluids, **dadurch gekennzeichnet, dass** die Zentrifugalpumpe einen elektromagnetischen Drehantrieb (1) umfasst, der gemäss einem der vorangehenden Ansprüche ausgestaltet ist, wobei der Rotor (3) des elektromagnetischen Drehantriebs (1) als Rotor (3) der Zentrifugalpumpe ausgestaltet ist.

10. Zentrifugalpumpe nach Anspruch 9, welche eine Pumpeneinheit (50) mit einem Pumpengehäuse (51) umfasst, das einen Einlass (52) und einen Auslass (53) für das zu fördernde Fluid umfasst, wobei der Rotor (3) im Pumpengehäuse (51) angeordnet ist, und eine Mehrzahl von Flügeln (54) zum Fördern des Fluids umfasst, wobei die Pumpeneinheit (50) derart ausgestaltet ist, dass die Pumpeneinheit (50) so in den Stator (2) einsetzbar ist, dass der magnetisch wirksame Kern (31) des Rotors (3) von den Statorpolen (21) umgeben wird.

11. Pumpeneinheit für eine Zentrifugalpumpe, **dadurch gekennzeichnet, dass** die Pumpeneinheit (50) für eine Zentrifugalpumpe (100) ausgestaltet ist, welche nach Anspruch 10 ausgestaltet ist.

12. Pumpeneinheit nach Anspruch 11, wobei das Pumpengehäuse (51) ein Basisteil (512) und einen Deckel (511) umfasst, welche dichtend miteinander verbunden sind, wobei der Auslass (53) des Pumpengehäuses (51) vollständig im Basisteil (512) angeordnet ist.

13. Pumpeneinheit nach einem der Ansprüche 11-12, wobei der Rotor (3) eine zentrale Bohrung (36) aufweist, welche sich in axialer Richtung (A) vollständig durch den Rotor (3) hindurch erstreckt.

14. Pumpeneinheit nach einem der Ansprüche 11-13, welche zum lösbaren Verbinden mit dem Stator (2) der Zentrifugalpumpe (100) nach Anspruch 9 ausgestaltet ist.

15. Pumpeneinheit nach einem der Ansprüche 11-14, wobei die Pumpeneinheit (50) als Einmalvorrichtung für den Einmalgebrauch ausgestaltet ist.
